(19) **Europäisches Patentamt / European Patent Office / Office européen des brevets**

(11) **EP 3 347 487 B1**

(12) **EUROPEAN PATENT SPECIFICATION**

(45) Date of publication and mention
of the grant of the patent:
**14.07.2021 Bulletin 2021/28**

(51) Int Cl.:
*C12Q 1/6883* (2018.01)  *A61K 35/545* (2015.01)
*C12N 5/0735* (2010.01)

(21) Application number: **16763789.1**

(22) Date of filing: **09.09.2016**

(86) International application number:
**PCT/EP2016/071256**

(87) International publication number:
**WO 2017/042309 (16.03.2017 Gazette 2017/11)**

(54) **NON-INVASIVE METHODS FOR ASSESSING GENETIC INTEGRITY OF PLURIPOTENT STEM CELLS**

NICHTINVASIVES VERFAHREN ZUR BEURTEILUNG DER GENETISCHEN INTEGRITÄT PLURIPOTENTER STAMMZELLEN

PROCÉDÉS NON INVASIFS POUR L'ÉVALUATION DE L'INTÉGRITÉ GÉNÉTIQUE DE CELLULES SOUCHES PLURIPOTENTES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **11.09.2015 EP 15306389**

(43) Date of publication of application:
**18.07.2018 Bulletin 2018/29**

(73) Proprietors:
• **INSERM (Institut National de la Santé et de la Recherche Médicale)**
**75013 Paris (FR)**
• **Université de Montpellier**
**34090 Montpellier (FR)**
• **Centre Hospitalier Universitaire de Montpellier**
**34000 Montpellier (FR)**

(72) Inventors:
• **DE VOS, John**
**34295 Montpellier Cedex 5 (FR)**
• **ASSOU, Said**
**34295 Montpellier Cedex 5 (FR)**
• **GIRAULT, Nicolas**
**34295 Montpellier Cedex 5 (FR)**

(74) Representative: **Regimbeau**
**La Coupole Sud**
**329, rue Léon Blum**
**34000 Montpellier (FR)**

(56) References cited:
**WO-A1-2014/202696   US-A1- 2015 031 030**

• **KRISTEN MARTINS-TAYLOR ET AL: "Recurrent copy number variations in human induced pluripotent stem cells", NATURE BIOTECHNOLOGY, vol. 29, no. 6, 1 June 2011 (2011-06-01), pages 488-491, XP055239098, US ISSN: 1087-0156, DOI: 10.1038/nbt.1890**
• **ELISA NÄRVÄ ET AL: "High-resolution DNA analysis of human embryonic stem cell lines reveals culture-induced copy number changes and loss of heterozygosity", NATURE BIOTECHNOLOGY, vol. 28, no. 4, 1 April 2010 (2010-04-01), pages 371-377, XP055239106, US ISSN: 1087-0156, DOI: 10.1038/nbt.1615**
• **WALTER D FUNK ET AL: "Evaluating the genomic and sequence integrity of human ES cell lines; comparison to normal genomes", STEM CELL RESEARCH, ELSEVIER, NL, vol. 8, no. 2, 1 October 2011 (2011-10-01), pages 154-164, XP028447661, ISSN: 1873-5061, DOI: 10.1016/J.SCR.2011.10.001 [retrieved on 2011-10-08]**
• **SETH M TAAPKEN ET AL: "Karotypic abnormalities in human induced pluripotent stem cells and embryonic stem cells", NATURE BIOTECHNOLOGY, vol. 29, no. 4, 1 April 2011 (2011-04-01), pages 313-314, XP055239110, US ISSN: 1087-0156, DOI: 10.1038/nbt.1835**

**(Cont. next page)**

- **MARGIT ROSNER ET AL: "Amniotic fluid stem cells and fetal cell microchimerism", TRENDS IN MOLECULAR MEDICINE, vol. 19, no. 5, 1 May 2013 (2013-05-01), pages 271-272, XP055239117, GB ISSN: 1471-4914, DOI: 10.1016/j.molmed.2013.01.001**
- **SAID ASSOU ET AL: "Recurrent Genetic Abnormalities in Human Pluripotent Stem Cells: Definition and Routine Detection in Culture Supernatant by Targeted Droplet Digital PCR", STEM CELL REPORTS, vol. 14, no. 1, 1 January 2020 (2020-01-01), pages 1-8, XP055663170, United States ISSN: 2213-6711, DOI: 10.1016/j.stemcr.2019.12.004**

**Description**

**FIELD OF THE INVENTION:**

The present invention relates generally to the fields of regenerative medicine. More specifically, the present invention relates to non-invasive methods and kits for determining the quality of pluripotent stem cells. More specifically, the present disclosure relates to noninvasive methods and kits for assessing genetic integrity of pluripotent stem cells in culture.

**BACKGROUND OF THE INVENTION:**

Human pluripotent stem cells (hPSC) research offers new tools to help understanding and treating diseases that affect diverse cells types in the body by producing human cells for transplantation or to enable drug discovery. PSC (isolated from the inner cell mass of discarded embryos, i.e. human embryonic stem cells (hESC), or derived from differentiated cells, i.e. induced pluripotent stem cells (iPSC)) have the remarkable capacity to expand rapidly. At a practical level, this means enough cells to manufacture thousands, and even hundred of thousands, of therapeutic cell doses can be generated from one cell line. Several clinical trials using differentiated derivatives of hESC have been or are currently ongoing: Geron Corporation (NCT01217008) has tested the safety of hESC-derived oligodendrocyte cells in patients with spinal cord injury. Advanced Cell Technology (ACT) has tested the safety of the hESC-derived retinal pigment epithelial (RPE) cellular therapy for Stargardt's Macular Dystrophy (SMD) (USA trial: NCT01345006; UK trial: NCT01469832; Korea trial: NCT01625559) and for Dry Age-Related Macular Degeneration (USA trial: NCT01344993; Korea trial: NCT01674829). Viacyte is testing the safety and efficacy of insulin producingcells in subjects with type I diabetes mellitus (USA trial: NCT02239354). Philippe Ménasché started testing (NCT02057900) the transplantation of human embryonic stem cell-derived progenitors in severe heart failure. Pfizer (NCT01691261) investigates the safety of using transplanted retinal cells derived from hESC to treat patients with advanced Stargardt disease. Finally, a study recently started in Japan, conducted by Masayo Takahashi from the RIKEN Institute, that is testing the safety of the transplantation of autologous induced pluripotent stem cell (iPSC)-derived retinal pigment epithelium (RPE) cell sheets in patients with exudative (wet-type) age-related macular degeneration (AMD).

All these clinical trials reveal that the biomedical potential is tremendous, but several practical matters remain to be resolved. One of the biggest concerns are the genetic abnormalities.

Genetic abnormalities are a serious concern for the use of hPSC for regenerative medicine. If hPSC clones display genetic abnormalities, these cells and their differentiated progeny might not be able to faithfully replicate the normal adult tissue physiology, and might even be a threat for the use of these cell in a clinical setting. It is thus mandatory to determine the cause and extent of genetic abnormalities in such cells. Genetic aberrations can be divided into two categories, those induced by cell culture, and those induced by the cell reprogramming process.

Human ESC are karyotypically normal at derivation; however, aneuploid hESC clones can appear during cell culture. Since 2004, several studies have reported that culture conditions used to amplify undifferentiated hPSC have a significant impact on chromosomal stability. Such chromosomal abnormalities are often recurrent. Gains of chromosomes 12 (most frequently 12p), 17 (particularly 17q), 20 or X have been often detected using standard cytogenetic procedures (G-banding) (Draper et al., 2004). An extensive study of 40 hESC lines in which 1163 karyotypes were analyzed concluded that 12.9% of the hESC culture displayed chromosomal aberrations (Taapken et al., 2011). Over the past five years, the resolution for genomic alteration detection was improved with array-based technologies (also called virtual karyotypes). Array-based comparative genomic hybridization (aCGH) or Single Nucleotide Polymorphism (SNP)-arrays have allowed the identification of small-size genomic aberrations and have revealed that the frequency of DNA alteration in hPSC could even be much higher than previously thought (Laurent et al., 2011; Narva et al., 2010). Among these small-size chromosomal changes, a recurrent copy number variant (CNV) located at chromosome 20q11.21 has been identified (Lefort et al., 2008; Spits et al., 2008). The 20q11.21 region is also amplified in a variety of cancers. Moreover it has been shown that the acquisition of 20q11.2 occurs at an early stage in cervical cancer. Point mutations also contribute to the adaptation process. More recently, whole exome or whole genome resequencing have provided unprecedented resolution for identifying single base-pair mutation in hPSCs (Cheng et al., 2012; Funk et al., 2012; Gore et al., 2011). The generation of iPS by cell reprogramming opens the way to other potential sources of mutations. Detailed analyses, by using CGH microarrays (Martins-Taylor and Xu, 2010; Pasi et al., 2011), SNP microarrays (Hussein et al., 2011; Laurent et al., 2011) or next-generation sequencing techniques (Gore et al., 2011; Ji et al., 2012) suggest that more subtle abnormalities, such as copy number variations (CNV) and mutations, occur in iPS cells at much higher frequency than originally thought. The exact load of mutations induced by cell reprogramming is however highly debated (Bai et al., 2013). Nevertheless, hiPS can also accumulate genetic alterations during cell culture. KRISTEN MARTINS-TAYLOR ET AL, NATURE BIOTECHNOLOGY, vol. 29, no. 6, 1 June 2011, pages 488-491, relates to recurrent copy number variations in human induced pluripotent stem cells.

These genetic abnormalities are a strong concern because any DNA mutation may be a step in a malignant transformation process. In addition, some abnormalities are highly recurrent, suggesting a strong selection pressure mediated by an increase in cell survival, cell proliferation or blockage of differentiation. These functional modifications may increase the susceptibility of PSC to malignant transformation and alter their expected therapeutic properties.

Pluripotent stem cells DNA integrity is mainly assessed by karyotype analysis. Other approaches have been tested to overcome the obvious resolution limitations of the classic karyotyping techniques, for example CGH arrays or SNP microarrays; however, there is no consensus on the method to use to discriminate between the really worrying, possibly carcinogenic mutations and the DNA modifications with no or barely any impact on the biological behavior of PSCs, or the simple polymorphisms. As DNA sequencing technologies and their resolution (whole genome maps at single-base resolution) are improving very fast and their price rapidly decreasing we can anticipate that one day routine analysis of PSC will rely on whole genome sequencing. However, each of these techniques have strong limitations. For instance, the classical karyotyping technique is time consuming, require the expertise of a cytogeneticist and is unable to detect abnormalities less than 5 Mb long. Microarray-based approaches require a core facility and bioinformaticians dedicated for the analysis. Finally, the high-throughput sequencing techniques such as NGS are not yet optimized for this use and the time necessary to process the data is long and also requires bioinformaticians.

Therefore, there is a strong need for a quick, inexpensive and non-invasive (without destroying the cells) methods, capable to detect the most recurrent abnormalities in hPSC.

## SUMMARY OF THE INVENTION:

The present invention is defined in the appended claims. The present disclosure relates to non-invasive methods and kits for determining the quality of pluripotent stem cell.

The present disclosure also relates to non-invasive methods and kits for assessing genetic integrity of pluripotent stem cell in culture.

## DETAILED DESCRIPTION OF THE INVENTION:

Pluripotent stem cells (PSC), that perpetuate by self-renewal but are able to differentiate into mature cells of particular tissues, are key tools for regenerative medicine. Regenerative medicine is a broad definition for innovative medical therapies that enable the body to repair, replace, restore and regenerate damaged or diseased cells, tissues and organs. But cell culture may result in epigenetic and genetic abnormalities that may alter the properties of stem cells or predispose them to tumor formation. With the rapid expansion of the use of PSC in the clinics, it is timely to improve tools to characterize pluripotent stem cells (PSC) during cell expansion and before batch release.

The inventors have determined a set of "hyper-recurrent sequences" in human pluripotent stem cells (hPSC) that are biomarkers for hPSC instability in culture (Table 1) and propose a rapid and easy-to-perform test that can be used to routinely assess stem cells during culture and prior to clinical use.

**Table 1: List of the 40 hyper-recurrent sequences.**

| Location represents the 5' end of amplification, based upon human genome build 37 (GRCh37/hgl9). | | | | | |
|---|---|---|---|---|---|
| **Sequence** | **Name** | **Chromosome** | **Start** | **End** | **Taille** |
| **S1** | Chr12:37868436-38954035 | chr12 | 37868436 | 38954035 | 1085599 |
| **S2** | Chr12:31248370-33127685 | chr12 | 31248370 | 33127685 | 1879315 |
| **S3** | Chr12:103681877-104694741 | chr12 | 103681877 | 104694741 | 1012864 |
| **S4** | Chr17:56720571-58041412 | chr17 | 56720571 | 58041412 | 1320841 |
| **S5** | Chr17:18387206- 20281117 | chr17 | 18387206 | 21243600 | 2856394 |
| **S6** | Chr17:6500001-10700000 | chr17 | 6500001 | 10700000 | 4199999 |
| **S7** | ChrX:6451571-7623882 | chrX | 6451571 | 7623882 | 1172311 |
| **S8** | Chr20:29846339-31316340 | chr20 | 29846339 | 31316340 | 1470001 |
| **S9** | chr20:29267955-30375868 | chr20 | 29267955 | 62166322 | 32898367 |
| **S10** | Chr1:201725542-203350641 | chr1 | 201725542 | 203350641 | 1625099 |
| **S11** | Chr1:144045189-145290292 | chr1 | 144045189 | 145290292 | 1245103 |

(continued)

| Location represents the 5' end of amplification, based upon human genome build 37 (GRCh37/hgl9). | | | | | |
|---|---|---|---|---|---|
| Sequence | Name | Chromosome | Start | End | Taille |
| S12 | Chr1:55908317-57681060 | chr1 | 55908317 | 57681060 | 1772743 |
| S13 | Chr7:135120003-136737366 | chr7 | 135120003 | 136737366 | 1617363 |
| S14 | Chr7:1-2800000 | chr 7 | 1 | 28000000 | 27999999 |
| S15 | Chr7:69817651-70852210 | chr7 | 69817651 | 70852210 | 1034559 |
| S16 | Chr5:69342002-70409630 | chr5 | 69342002 | 70496687 | 1154685 |
| S17 | Chr5:133166096-135117724 | chr5 | 133166096 | 135117724 | 1951628 |
| S18 | Chr6:162765665-164166909 | chr6 | 162765665 | 164166909 | 1401244 |
| S19 | Chr6:119517636-121231501 | chr6 | 119517636 | 121231501 | 1713865 |
| S20 | Chr8:23538410-24752559 | chr8 | 23538410 | 24752559 | 1214149 |
| S21 | Chr8:144247611-145708651 | chr8 | 144247611 | 145708651 | 1461040 |
| S22 | Chr9:44391266-46306410 | chr9 | 44391266 | 46306410 | 1915144 |
| S23 | Chr9:68317831-69630327 | chr9 | 68317831 | 69978010 | 1660179 |
| S24 | chr10:46388145-47479792 | chr10 | 46388145 | 47484886 | 1096741 |
| S25 | chr10:64199868-65487432 | chr10 | 64199868 | 65487432 | 1287564 |
| S26 | Chr11:48970262-51052887 | chr11 | 48970262 | 51052887 | 2082625 |
| S27 | Chr13:108583470-110381343 | chr13 | 108583470 | 110381343 | 1797873 |
| S28 | Chr14:19377573-20399480 | chr14 | 19377573 | 20399480 | 1021907 |
| S29 | Chr16:32049230-33693642 | chr16 | 32049230 | 34079200 | 2029970 |
| S30 | Chr16:32000261-33537523 | chr16 | 32000261 | 33736180 | 1735919 |
| S31 | Chr15:18828463-19840461 | chr15 | 18828463 | 20095423 | 1266960 |
| S32 | chr15:20935078-22210804 | chr15 | 20935078 | 22210804 | 1275726 |
| S33 | Chr18:57994812-59707736 | chr18 | 57994812 | 59707736 | 1712924 |
| S34 | Chr2:90134268-91622003 | chr2 | 90134268 | 91622003 | 1487735 |
| S35 | Chr2:89133113-90135873 | chr2 | 89133113 | 90211593 | 1078480 |
| S36 | Chr4:123413720-124418903 | chr4 | 123413720 | 124418903 | 1005183 |
| S37 | Chr4:93113276-94193881 | chr4 | 93113276 | 94193881 | 1080605 |
| S38 | Chr19:45074171-46122773 | chr19 | 45074171 | 46122773 | 1048602 |
| S39 | Chr3:36600001-38600000 | chr3 | 36600001 | 38600000 | 1999999 |
| S40 | Chr21:11084669-14603577 | chr21 | 11084669 | 14642464 | 3557795 |

Accordingly, the present disclosure relates to an *in vitro* non invasive method for determining the quality of pluripotent stem cell comprising the steps of: i) providing a culture sample where the pluripotent stem cell is grown, ii) extracting nucleic acids from the sample and iii) determining the presence and/or level of at least one genetic abnormality in the nucleic acid extraction.

As used herein the term "pluripotent stem cell" or "PSC" has its general meaning in the art and refers to pluripotent cell such as embryonic stem cell (ESC) and induced pluripotent stem cell (iPSC), which is capable of differentiating into any cell type in the human body. The term "Pluripotent" refers to cell that is capable of differentiating into one of a plurality of different cell types, although not necessarily all cell types. Cells used in the invention include but are not limited to cardiomyocytes and progenitors thereof; neural progenitor cells; pancreatic islet cells, particularly pancreatic β-cells; hematopoietic stem and progenitor cells; mesenchymal stem cells; and muscle satellite cells. The method of the invention is applicable to pluripotent stem cells but is also applicable to other stem cells, germinal or somatic cells (e.g., Mesenchymal stem cells (MSC), oocyte, embryo, fibroblasts...).

By "determining the quality of pluripotent stem cell" it is meant that the method of the invention aims at determining whether pluripotent stem cell bear a genetic abnormality or a specific sequence in the context of regenerative medicine. The method of the invention allows the assessment of genetic integrity and genetic stability of pluripotent stem cell in culture.

As used herein the term "genetic abnormality" refers to any event that can exist in the genome of an individual and pluripotent stem cell that can give rise to cause a phenotypic disease and lethality. Genetic abnormalities include but are not limited to trisomy, translocation, quadrisomy, aneuploidy, partial aneuploidy, monosomy, karyotype abnormality, isodicentric chromosome, isochromosome, inversion, insertion, duplication, deletion, copy number variation (CNV), chromosome translocation, Single nucleotide variation (SNV), and Loss of heterozygosity (LOH). Typically, the term "genetic abnormality" refers to hyper-recurrent sequences such as described in Table 1.

The term "culture sample" refers to culture supernatant, culture medium and cells in suspension in the culture.

As used herein the term "nucleic acid" has its general meaning in the art and refers to a coding or non coding nucleic sequence. Nucleic acids include DNA (deoxyribonucleic acid) and RNA (ribonucleic acid). Example of nucleic acid thus include but are not limited to DNA, mRNA, tRNA, rRNA, tmRNA, miRNA, piRNA, snoRNA, and snRNA. The term "nucleic acids" also relates to free nucleic acids (fNA) (originate form the nucleus of the cells or from the mitochondrial compartment of the cells) such as cell free DNA, free RNA molecules, microRNAs, and long non-coding RNA. By "free nucleic acid" it is meant that the nucleic acid is released by the pluripotent stem cells and is present in the culture medium wherein the pluripotent stem cells are grown.

Any methods well known in the art may be used by the skilled artisan in the art for extracting the free cell nucleic acid from the prepared sample. For example, the method described in the example may be used.

In a particular embodiment, the method of the invention comprises the steps of i) determining the presence of at least one hyper-recurrent sequence in the nucleic acid extraction, and ii) concluding that the pluripotent stem cells bears a genetic abnormality when at least one hyper-recurrent sequence is detected.

Typically, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 hyper-recurrent sequences may be selected from table 1.

Determination of the presence and the level of hyper-recurrent sequence in the nucleic acid extraction can be performed by a variety of techniques well known in the art. In a particular embodiment, droplet digital-PCR "ddPCR" may be performed for determining the presence and the level of hyper-recurrent sequence in the nucleic acid extraction. ddPCR refers to a method or device used therein that allows for the quantification of DNA sequences in a supernatant or culture medium.

Determination of the presence and the level of hyper-recurrent sequence in the nucleic acid extraction can also be performed by techniques such as Fluidigm, quantitative PCR, high-throughput paired-end sequencing, next-generation sequencing, and capillary electrophoresis.

Typical techniques for detecting a hyper-recurrent sequence in a nucleic acid in particular DNA or mRNA include but are not limited restriction fragment length polymorphism, hybridisation techniques, sequencing, exonuclease resistance, microsequencing, solid phase extension using ddNTPs, extension in solution using ddNTPs, oligonucleotide assays, methods for detecting single nucleotide polymorphism such as dynamic allele-specific hybridisation, ligation chain reaction, mini-sequencing, DNA "chips", allele-specific oligonucleotide hybridisation with single or dual-labelled probes merged with PCR or with molecular beacons, and others.

Typically, hyper-recurrent sequences are detected after amplification. For instance, the isolated RNA may be subjected to coupled reverse transcription and amplification, such as reverse transcription and amplification by polymerase chain reaction (RT-PCR), using specific oligonucleotide primers that are specific for a hyper-recurrent sequence or that enable amplification of a region containing the hyper-recurrent sequence. According to a first alternative, conditions for primer annealing may be chosen to ensure specific reverse transcription (where appropriate) and amplification; so that the appearance of an amplification product be a diagnostic of the presence of a particular hyper-recurrent sequence. Otherwise, RNA may be reverse-transcribed and amplified, or DNA may be amplified, after which a hyper-recurrent sequence may be detected in the amplified sequence by hybridization with a suitable probe or by direct sequencing, or any other appropriate method known in the art. For instance, a cDNA obtained from RNA may be cloned and sequenced to identify a hyper-recurrent sequence.

In particular sequencing represents an ideal technique that can be used in the context of the present invention. The one skilled in the art is familiar with several methods for sequencing of polynucleotides. These include, but are not limited to, Sanger sequencing (also referred to as dideoxy sequencing) and various sequencing-by-synthesis (SBS) methods as reviewed by Metzger (Metzger ML 2005, Genome Research 1767), sequencing by hybridization, by ligation (for example, WO 2005/021786), by degradation (for example, U.S. Patent Nos. 5,622,824 and 6,140,053), nanopore sequencing. Preferably in a multiplex assay deep sequencing is preferred. The term "deep sequencing" refers to a method of sequencing a plurality of nucleic acids in parallel. See e.g., Bentley et al, Nature 2008, 456:53-59. The leading commercially available platforms produced by Roche/454 (Margulies et al., 2005a), Illumina/Solexa (Bentley et al., 2008), Life/APG (SOLiD) (McKernan et al., 2009) and Pacific Biosciences (Eid et al., 2009) may be used for deep sequencing. For example, in the 454 method, the DNA to be sequenced is either fractionated and supplied with adaptors or segments of DNA can be PCR-amplified using primers containing the adaptors. The adaptors are nucleotide 25-mers required for binding to the DNA Capture Beads and for annealing the emulsion PCR Amplification Primers and the Sequencing Primer. The DNA fragments are made single stranded and are attached to DNA capture beads in a manner that allows only one DNA fragment to be attached to one bead. Next, the DNA containing beads are emulsified in a water- in-oil mixture resulting in microreactors containing just one bead. Within the microreactor, the fragment is PCR-amplified, resulting in a copy number of several million per bead. After PCR, the emulsion is broken and the beads are loaded onto a pico titer plate. Each well of the pico-titer plate can contain only one bead. Sequencing enzymes are added to the wells and nucleotides are flowed across the wells in a fixed order. The incorporation of a nucleotide results in the release of a pyrophosphate, which catalyzes a reaction leading to a chemiluminescent signal. This signal is recorded by a CCD camera and a software is used to translate the signals into a DNA sequence. In the Illumina method (Bentley (2008)), single stranded, adaptor-supplied fragments are attached to an optically transparent surface and subjected to "bridge amplification". This procedure results in several million clusters, each containing copies of a unique DNA fragment. DNA polymerase, primers and four labeled reversible terminator nucleotides are added and the surface is imaged by laser fluorescence to determine the location and nature of the labels. Protecting groups are then removed and the process is repeated for several cycles. The SOLiD process (Shendure (2005)) is similar to 454 sequencing, DNA fragments are amplified on the surface of beads. Sequencing involves cycles of ligation and detection of labeled probes. Several other techniques for high-throughput sequencing are currently being developed. Examples of such are The Helicos system (Harris (2008)), Complete Genomics (Drmanac (2010)) and Pacific Biosciences (Lundquist (2008)). As this is an extremely rapidly developing technical field, the applicability to the present invention of high throughput sequencing methods will be obvious to a person skilled in the art.

Determining the expression level of a nucleic acid (in particular a gene, miRNA, snRNA, and snoRNA) may be assessed by any of a wide variety of well-known methods. Typically the prepared nucleic acid can be used in hybridization or amplification assays that include, but are not limited to, Southern or Northern analyses, polymerase chain reaction analyses, such as quantitative PCR (TaqMan), and probes arrays such as GeneChip(TM) DNA Arrays (AFF YMETRIX). Advantageously, the analysis of the expression level of a nucleic acid involves the process of nucleic acid amplification, e. g., by RT-PCR (the experimental embodiment set forth in U. S. Patent No. 4,683, 202), ligase chain reaction (BARANY, Proc. Natl. Acad. Sci. USA, vol.88, p: 189-193, 1991), self sustained sequence replication (GUATELLI et al., Proc. Natl. Acad. Sci. USA, vol.57, p: 1874-1878, 1990), transcriptional amplification system (KWOH et al., 1989, Proc. Natl. Acad. Sci. USA, vol.86, p: 1173-1177, 1989), Q-Beta Replicase (LIZARDI et al., Biol. Technology, vol.6, p: 1197, 1988), rolling circle replication (U. S. Patent No. 5,854, 033) or any other nucleic acid amplification method, followed by the detection of the amplified molecules using techniques well known to those of skill in the art. Real-time quantitative or semi-quantitative RT-PCR is preferred. In a particular embodiment, the determination comprises hybridizing the sample with selective reagents such as probes or primers and thereby detecting the presence, or measuring the amount of the nucleic acid. Hybridization may be performed by any suitable device, such as a plate, microtiter dish, test tube, well, glass, column, and so forth. Nucleic acids exhibiting sequence complementarity or homology to the nucleic acid of interest herein find utility as hybridization probes or amplification primers. It is understood that such nucleic acids need not be identical, but are typically at least about 80% identical to the homologous region of comparable size, more preferably 85% identical and even more preferably 90-95% identical. In certain embodiments, it will be advantageous to use nucleic acids in combination with appropriate means, such as a detectable label, for detecting hybridization. A wide variety of appropriate indicators are known in the art including, fluorescent, radioactive, enzymatic or other ligands (e.g. avidin/biotin). The probes and primers are "specific" to the nucleic acid they hybridize to, *i.e.* they preferably hybridize under high stringency hybridization conditions (corresponding to the highest melting temperature -Tm-, *e.g.,* 50 % formamide, 5x or 6x SCC. 1x SCC is a 0.15 M NaCl, 0.015 M Na-citrate). Many quantification assays are commercially available from Qiagen (S.A. Courtaboeuf, France) or Applied Biosystems (Foster City, USA). Expression level of the nucleic acid may be expressed as absolute expression profile or normalized expression profile. Typically, expression profiles are normalized by correcting the absolute expression profile of the nucleic acid of interest by comparing its expression to the expression of a nucleic acid that is not a relevant, e.g., a housekeeping mRNA that is constitutively expressed. Suitable mRNA for normalization include housekeeping mRNAs such as the U6, U24, U48 and S18. This normalization allows the comparison of the expression profile in one sample, e.g., a patient sample, to another sample, or between samples from different sources.

Probe and or primers are typically labelled with a detectable molecule or substance, such as a fluorescent molecule, a radioactive molecule or any others labels known in the art. Labels are known in the art that generally provide (either directly or indirectly) a signal. The term "labelled" is intended to encompass direct labelling of the probe and primers by coupling (i.e., physically linking) a detectable substance as well as indirect labeling by reactivity with another reagent that is directly labeled. Examples of detectable substances include but are not limited to radioactive agents or a fluorophore (e.g. fluorescein isothiocyanate (FITC) or phycoerythrin (PE) or Indocyanine (Cy5)).

The method of the disclosure is particularly suitable for determining the quality of pluripotent stem cell culture, and then isolating pluripotent stem cell free from genetic abnormalities. The method as above described is particularly suitable for avoiding destruction of pluripotent stem cell culture containing pluripotent stem cell free from genetic abnormalities which may be isolated and cultured.

Accordingly, the present disclosure relates to a method for isolating a pluripotent stem cell free from genetic abnormalities comprising the steps of:

i) determining the level of hyper-recurrent sequences in a pluripotent stem cell culture by performing the method according to the invention,
ii) comparing the level determined at step i) with a reference value,
iii) concluding that the pluripotent stem cell culture contains pluripotent stem cell free from genetic abnormalities when the level determined at step i) is different from the reference value,
iv) and isolating said pluripotent stem cell free from genetic abnormalities.

The step of isolating pluripotent stem cell can be performed by a variety of techniques well known in the art such as fluidigm technique.

In a particular embodiment, the reference value is a threshold value or a cut-off value that can be determined experimentally, empirically, or theoretically. A threshold value can also be arbitrarily selected based upon the existing experimental conditions, as would be recognized by a person of ordinary skilled in the art. The threshold value has to be determined in order to obtain the optimal sensitivity and specificity according to the function of the test and the benefit/risk balance (clinical consequences of false positive and false negative). Typically, the optimal sensitivity and specificity (and so the threshold value) can be determined using a Receiver Operating Characteristic (ROC) curve based on experimental data. Preferably, the person skilled in the art may compare the nucleic acid levels (obtained according to the method of the invention) with a defined threshold value. In one embodiment of the present invention, the threshold value is derived from the nucleic acid levels (or ratio, or score) determined in pluripotent stem cell culture bearing genetic abnormalities. Furthermore, retrospective measurement of the nucleic acid levels (or ratio, or scores) in properly banked historical pluripotent stem cells cultures may be used in establishing these threshold values.

The method of the invention is particularly suitable for reaching a clinical decision. As used herein the term "clinical decision" refers to any decision to take or not take an action that has an outcome that affects the health or survival of the subject. In particular, in the context of the invention, a clinical decision refers to a decision to transfer, graft, transplant or not the pluripotent stem cell to the subject. A clinical decision may also refer to a decision to conduct further testing, to take actions to mitigate an undesirable phenotype. In particular, the method as above described will thus help clinician to avoid the transfer to the subject of pluripotent stem cell bearing genetic abnormalities. The method as above described is also particularly suitable for avoiding contamination of the subject by pluripotent stem cell bearing genetic abnormalities, avoiding the development of diseases such as malignancies caused by the transfer of pluripotent stem cell bearing genetic abnormalities to the subject. The method as above described is also particularly suitable for treating a subject in need thereof by administering pluripotent stem cell without side effects.

As used herein, the term "subject" denotes a mammal. Typically, a subject according to the invention refers to any subject (preferably human) in need of regenerative treatment using pluripotent stem cell transplantation. The term "subject" also refers to other mammals such as primates, dogs, cats, pigs, cows, or mouse. In a particular embodiment, the term "subject" refers to a subject afflicted with or susceptible to be afflicted with diseases in need of regenerative treatment using pluripotent stem cell transplantation such as spinal cord injury, Stargardt's Macular Dystrophy (SMD), Dry Age-Related Macular Degeneration, type I diabetes mellitus, cardiovascular disorders such as heart failure, advanced Stargardt disease, exudative (wet-type) age-related macular degeneration (AMD), muscular dystrophies, neurologic and retinal diseases, liver disease and diabetes.

Accordingly, the method of the invention allows the assessment of the ability of pluripotent stem cell to perform a healthy transfer, graft or transplantation to a subject. The method of the invention allows genetic testing and selection of pluripotent stem cell that is able to be transferred, grafted or transplanted to a subject.

The pluripotent stem cell selected by performing the method of the invention and differentiated cells derived therefrom find use in regenerative medicine. The term "regenerative medicine" has its general meaning in the art and refers to the regenerative treatment relating to process of creating living, functional cells and tissues to repair or replace cells, tissue or organ function lost due to age, disease, damage, or congenital defects.

Accordingly, the present disclosure also relates to a method for the transplantation of pluripotent stem cell or differentiated cells derived therefrom to a subject in need of regenerative treatment comprising the steps of: i) performing the method according to the invention, ii) selecting pluripotent stem cell free from genetic abnormalities, and iii) administering the pluripotent stem cell selected at step ii) or differentiated cells derived therefrom to said subject.

In a further aspect, the methods of the disclosure are particularly suitable for treating a disease in a subject in need of regenerative treatment using pluripotent stem cell transplantation with a minimum of risk of genetic abnormality transfer. The methods of the invention are also suitable for treating a disease in a subject in need of regenerative treatment using pluripotent stem cell transplantation with a minimum of risk of developing diseases such as malignancies caused by the transfer of pluripotent stem cell bearing a genetic abnormality.

Accordingly the disclosure also relates to a method for treating a disease in a subject in need of regenerative treatment comprising the steps of: i) performing the method according to the invention, ii) selecting pluripotent stem cell free from genetic abnormalities, and iii) administering the pluripotent stem cell selected at step ii) or differentiated cells derived therefrom to said subject.

In a further aspect, the present disclosure relates to a method for enhancing response to regenerative treatment in a subject in need thereof comprising the steps of: i) performing the method according to the invention, ii) selecting pluripotent stem cell free from genetic abnormalities, and iii) administering the pluripotent stem cell selected at step ii) or differentiated cells derived therefrom to said subject.

The disclosure also relates to a kit for performing the methods as above described, wherein said kit comprises means for determining the presence and/or level of at least one genetic abnormality in the nucleic acid extraction. Typically, the kits include probes, primers, macroarrays or microarrays as above described. For example, the kit may comprise a set of probes as above defined, and that may be pre-labelled. Alternatively, probes may be unlabelled and the ingredients for labelling may be included in the kit in separate containers. The kit may further comprise hybridization reagents or other suitably packaged reagents and materials needed for the particular hybridization protocol, including solid-phase matrices, if applicable, and standards. Alternatively the kit of the invention may comprise amplification primers (e.g. stem-loop primers) that may be pre-labelled or may contain an affinity purification or attachment moiety. The kit may further comprises amplification reagents and also other suitably packaged reagents and materials needed for the particular amplification protocol. The kit may further comprises means necessary to determine if amplification has occurred. The kit may also include, for example, PCR buffers and enzymes; positive control sequences, reaction control primers; and instructions for amplifying and detecting the specific sequences.

The invention will be further illustrated by the following figures and examples. However, these examples and figures should not be interpreted in any way as limiting the scope of the present invention.

**FIGURES:**

Figure 1: Schematic illustration of the workflow. Envisioned use of a genomic analysis of supernatant to qualify the hPSC in culture. hPSC supernatant is collected and total cfDNA is extracted. Then PCR is performed. The results are then analyzed by bioinformatics to detect the biomarkers in the cfDNA.

Figure 2: Recurrence of hPSC genetic abnormalities collected in SEAdb. Color gradient: # studies; bubble size: length of the genomic region; Y: recurrence score; X: chromosome.

Figure 3: For the 21 chromosomes that harbor most genetic alterations > 1 Mb, the 40 sets of sequences of Table 1 (Sondes: S1-S40) cover 93,5 % of chromosomal abnormalities.

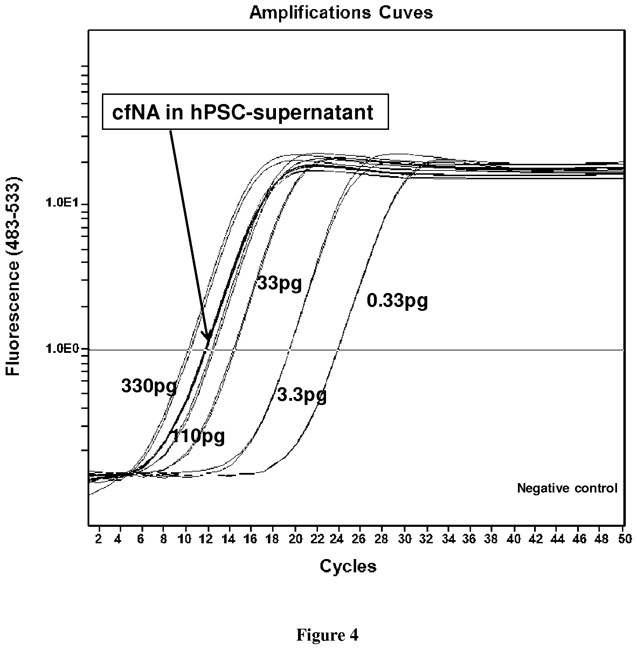

Figure 4: Detection and quantification of the cfNA in hPSC-supernatant samples. Human ALU-repeats sequence amplification was evaluated in two hPSC-supernatant and using DNA from human foreskin fibroblasts at five concentrations as control (330pg, 110pg, 13pg, 3.3pg and 0.33pg). QPCR experiments were performed on Roche LC480. Fluorescence was acquired at each cycle and plotted against the cycle number. The increasing amount of the measured fluorescence is proportional to the amount of PCR product generated during the reaction. The measured cfNA concentration in hPSC is between (330pg and 110pg).

Figure 5: Supernatant-based detection of trisomy 20. A. Representative abnormal karyotypes in the two hPSC lines: HD291 (47,XY,+12) (left panel) and HD129 (47,XY,+20) (right panel). B. ddPCR quantification of trisomy 20 in the two hPSC lines and there supernatants using a specific hyper-recurrent sequence for only trisomy 20. The copy number plot, with precise triplicate well, revealed the presence of trisomy 20 only in abnormal hPSC cells HD129 and their supernatant but not in HD291. All error bars generated by QuantaSoft™ software represent the 95% confidence interval.

Figure 6: High sensitivity of the QX200 system allows quantification of trisomy 20 in the hPSC-supernatant using specific hyper-recurrent sequence. Sample concentrations are plotted as copies/μl.

**EXAMPLES:**

**EXAMPLE 1:**

**Methods:**

**hPSC culture and supernatant collection**

Human PSC (hESC or iPSC) were cultured in 35-mm wells on Geltrex™ in presence of xeno-free and completely defined medium (Essential 8™ Medium). Cells were dissociated mechanically and grown in bulk culture or dissociated enzymatically and adapted to single cell passage. The medium was renewed every day. hPSC-free media were incubated as controls. One ml of supernatant (hPSC-conditioned media) from each well were collected just before routine passage of PSC and immediately frozen into sterile, DNA-, DNase-, RNase-, polymerase chain reaction (PCR) inhibitors-free tubes and stored at -80°C until nucleic acid purification. Appropriate precautions were taken to prevent contamination of samples by extraneous DNA.

**Nucleic acid purification**

Nucleic acid was extracted from 200 $\mu$l of supernatant by using the QIAmp DNA Mini Blood Kit (Qiagen, Hilden, Germany) according to the manufacturing protocol. Briefly, 20 $\mu$l Proteinase K and 200 $\mu$l Buffer AL were added to each supernatant. After pulse vortexing for 15 s, the lysis mixture was incubated at 56°C for 10 min in eppendorf tube (1.5 ml). The highly denaturing conditions at elevated temperatures favored the complete release of nucleic acids from any bound proteins. After adding 200 $\mu$l cold ethanol (100%) to the lysate, the sample was transferred onto a QIAamp Mini column. Cell-free nucleic acid was adsorbed onto the membrane as the lysate was drawn through by centrifugation at 6000g for 1 min. Contaminants were efficiently washed away during two wash steps (in Buffer AW1 and Buffer AW2). Finally, Cell-free nucleic acid was eluted in 30 $\mu$l Buffer AE and stored at -20°C.

**Quantification of cell-free nucleic acid (cfNA)**

The concentration of cfNA in each supernatant was assessed relative to the corresponding concentration of ALU-115 PCR product that was determined by quantitative PCR approach (LC480, Roche). For this purpose, one $\mu$l of each cfNA elute sample, was added to a reaction mixture containing commercially available 2X LightCycler480 SYBR Green I master mix (Roche Applied Science, Germany) and 0.25 $\mu$M of forward and reverse ALU-primers as described in Umetani et al. (2006) in a total volume of 10 $\mu$L. Reactions were set up in 96-white-well plates (Eppendorf) by means of EpMotion 5070 Liquid Handling Workstation (Eppendorf). All reactions were performed in triplicate. A negative control (RNAse/DNAse free water) was included in each run. The cfNA concentration in supernatant was determined using a standard curve obtained by successive dilutions of genomic nucleic acids extracted directly from hPSC.

**CNV detection in cfNA using the digital droplet PCR system (ddPCR)**

The ddPCR assay was performed as described previously (Abyzov et al. 2002). Briefly, the ddPCR workflow consists of setting up reactions, making droplets, thermal cycling and running on the droplet reader according to Bio-Rad instructions (Bio-Rad QX200 system). ddPCR makes use of fluorescently labeled internal hybridization probes (TaqMan probes) for detection of the CNV in cfNA. The reaction is normally set up using one primer pair targeted for the region of interest (for instance: CNV-ID1, dHsaCP2506319) and a second primer pair targeted for any standard reference gene (for instance: RPP30, dHsaCP2500350). The two primers (Target and reference) are labeled with different fluorophores (FAM and HEX). An input of cfNA from each supernatant was added to the TaqMan PCR reaction mixture. Such reaction mixture included ddPCR Supermix No dUTP (Bio-Rad, Ref: 1863023) and primers in a final volume of 20 $\mu$l. Each assembled ddPCR reaction mixture was then loaded into the sample well of an eight-channel disposable droplet generator cartridge (Bio-Rad, Ref:1864008). A volume of 60 $\mu$L of droplet generation oil (Bio-Rad, Ref:1863005) was loaded into the oil well for each channel. The cartridge was placed into the droplet generator (Bio-Rad). The cartridge was removed from the droplet generator, where the droplets that collected in the droplet well were then manually transferred with a multichannel pipet to a 96-well PCR plate. The plate was heat-sealed with a foil seal and then placed on a conventional thermal cycler and amplified to the end-point (40-50 cycles). Using microfluidic technology, the reaction mix is partitioned into spherical droplets composed of an oil surface and an aqueous core containing the PCR reaction mix. The droplets are subjected to thermal cycling. After amplification, the fluorescence of each droplet is read in succession by a droplet reader. Droplets that contain the target region of interest or reference will fluoresce in the corresponding channel (positive droplets), while those without target will not (negative droplets). The counts of positive and negative droplets for each target are related to the target's concentration in the sample by the Poisson distribution.

### Results:

Pluripotent stem cells (PSC), that perpetuate by self-renewal but are able to differentiate into mature cells of particular tissues, are key tools for regenerative medicine. Regenerative medicine is a broad definition for innovative medical therapies that enable the body to repair, replace, restore and regenerate damaged or diseased cells, tissues and organs. But cell culture may result in epigenetic and genetic abnormalities that may alter the properties of stem cells or predispose them to tumor formation. With the rapid expansion of the use of PSC in the clinics, it is timely to improve tools to characterize pluripotent stem cells (PSC) during cell expansion and before batch release.

Currently, there is no reliable commercially available genetic and non-invasive procedure for evaluation of genetic integrity of pluripotent stem cells in culture. The present invention relates to a method for assessing the genomic integrity of hPSC in culture, comprising a step of detection of genetic abnormalities in DNA present in supernatant collected during propagation of PSC in culture.

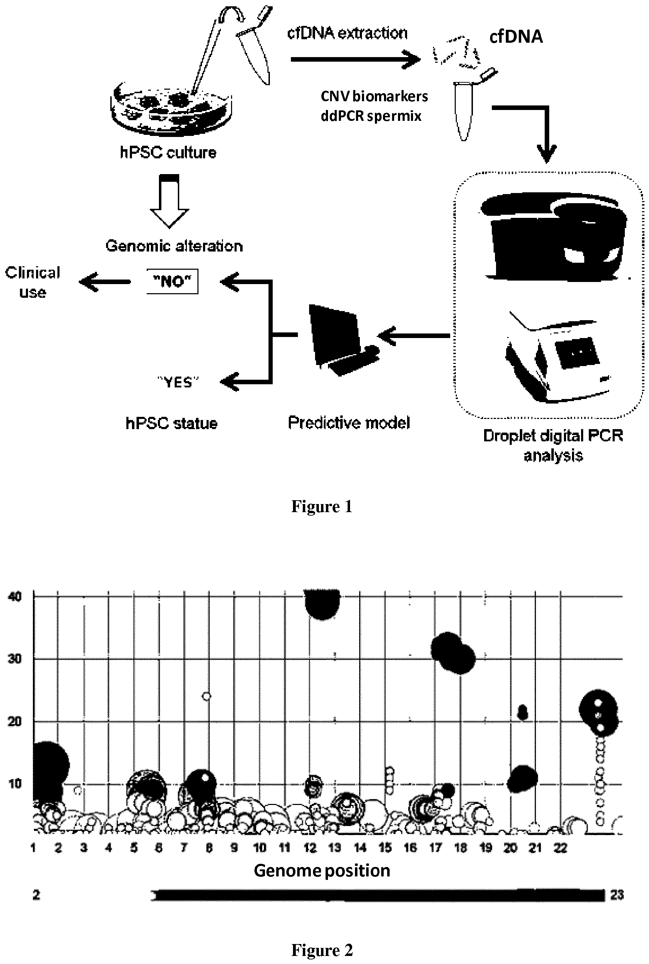

The inventors have determined as set of "hyper-recurrent sequences" in hPSC that are biomarkers for hPSC instability in culture (Table 1) and propose a rapid and easy-to-perform test that can be used to routinely assess stem cells during culture and prior to clinical use (Figure 1).

### Recurrent genetic alterations occurring during hPSC culture.

The inventors have developed a database "SEAdb" dedicated to the visualization of all types of genomic abnormalities, obtained by karyotype, FISH, microarray analysis (SNP, aCGH) or NGS. SEAdb can be accessed via the following link: seadb.org (login: seadb and pwd: SEAdb). The inventors have gathered abnormalities for more 400 000 abnormalities and variants.

The inventors showed that the most recurrent genetic alterations occurring during hPSC culture are karyotype abnormalities and copy number variations (CNVs) > 1 Mb (Figure 2).

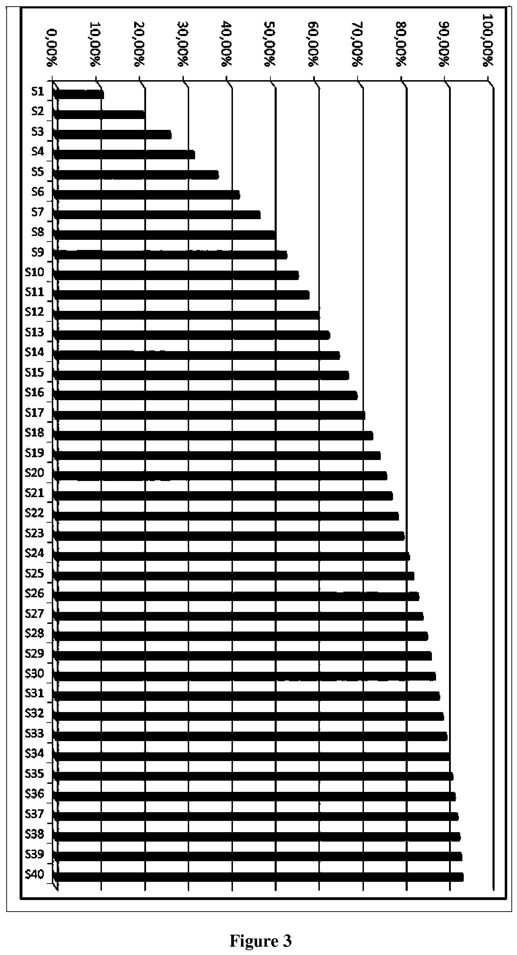

By contrast, smaller genetic abnormalities such as mutations and indels, have almost not recurrent. 1171 genetic alterations > 1 Mb are present in SEAdb. The inventors designated a recurrency score that helps us to identify the positions on the genome that are most prone to genome modification induced by PSC culture. For example, for the 21 chromosomes that harbor most genetic alterations > 1 Mb, the inventors showed that the 40 sets of sequences of Table 1 (Sondes: S1-S40) cover 93,5 % of chromosomal abnormalities (Figure 3).

### Cell culture supernatant as source for DNA to detect pathogenic sequences

A major constraint to assess the genome integrity of stem cells in culture is the need to destroy a sample of the culture to perform the test. Therefore, the inventor proposes that genome integrity can be carried out on the cell culture supernatant.

Indeed, cell culture supernatant contain cell-free DNA (cfDNA) that are doublestranded molecules with lower molecular weight than genomic DNA, in the form of short fragments (between 70 and 200 base pairs in length) or long fragments up to 21 kb. The mechanisms of cfDNA release are poorly known, but it has been suggested that necrosis, apoptosis, phagocytosis or active release may play a role (Choi et al., 2005; Gahan et al., 2008; Stroun et al., 2001).

CfDNA is present in the serum or plasma and used for non-invasive testing to detect chromosomal abnormalities (Hui and Bianchi, 2013). It was demonstrated that specific fetal aneuploidies, such as trisomy 13, 18 or 21, can be detected in cell-free fetal DNA from maternal serum samples (Dan et al., 2012; Fairbrother et al., 2013; Nicolaides et al., 2014). Moreover, fetal cfDNA in maternal plasma is also used to detect pathogenic copy number variations (CNV) using target region capture sequencing (Ge et al., 2013).

Based on the finding that the cfDNA are released in different fluids (serum, plasma) and can be used to detect pathogenic CNV, the inventors propose the use of DNA present in supernatant as source to detect pathogenic CNV and to perform a non-invasive analysis of the hPSC avoiding the cell destruction.

In order to evaluate a possible exogenous source of DNA in the stem cell supernatant, the inventor use quantitative real-time PCR of ALU repeats (Umetani et al., 2006). Quantification by ALU-qPCR of total cfDNA (triplicates) in two supernatant from two hESC showed unambiguously that the cfDNA is detected in all tested samples and the measured cfDNA concentration is between (330pg and 110pg) (Figure 4).

These results demonstrate that the hPSC-supernatant contains cell-free DNA (cfDNA), presumably resulting from the release of genetic material from dead cells, and floating live cells. The detection of cfDNA released in hPSC supernatant represents a yet unexplored tool to facilitate genetic abnormality evaluation using sequence-biomarkers.

The term "culture medium" relates to a nutrient solution for the culturing, growth or proliferation of cells. The term "cell culture" refers to cells which are maintained, cultivated or grown in an artificial in vitro environment.

The term "CNV" relates to alterations of the DNA of a genome that results in the cell having an abnormal or, for certain genes, a normal variation in the number of copies of one or more sections of the DNA. CNVs correspond to relatively large regions of the genome that have been deleted (fewer than the normal number) or duplicated (more than the normal number) on certain chromosomes.

**REFERENCE EXAMPLE 2:**

**Methods:**

**Karyotyping**

Human pluripotent stem cells were dissociated with TryPLE Select (Life Technologies) and grown for 3 days to reach mid-exponential phase. Then, single cells were incubated with the 1/10,000 KaryoMAX® Colcemid™ (Life Technologies) for 90 min for metaphase arrest before hypotonic swelling with 0.075 M KCl solution at 37°C for 20 min and three successive fixations in ice-cold methanol/glacial acetic acid (3:1, vol/vol). Twenty microliters of nuclei suspension were dropped on glass slides, air dried at 18.4°C and 60% humidity, and rehydrated in water for 5 min before denaturation in EARLE orange or 10× EBSS at 87°C for 55 min. Slides were then rinsed in cold water and stained with 3% GIEMSA for 3 min, rinsed five times, and air dried. Spectral microscopy and analysis were carried out using the Metafer Slide Scanning Platform (MetaSystem).

**Analysis of ddPCR data and statistics**

The number of droplets recording fluorescence for the target-specific assay (dHsaCP2506319) was compared to the count obtained for the reference-specific assay (dHsaCP2500350). Final copy numbers were calculated employing the manufacturer's QuantaSoft Software (Bio-Rad, CA, USA) by applying Poisson statistics:

$$\lambda = -\ln(1 - p)$$

Where "$\lambda$" is the average number of copies per droplet and "p" is the ratio of positive droplets to the total number of droplets.

**Results:**

**Evaluation of genetic integrity in hPSC-supernatant using ddPCR approach: application in routine screening**

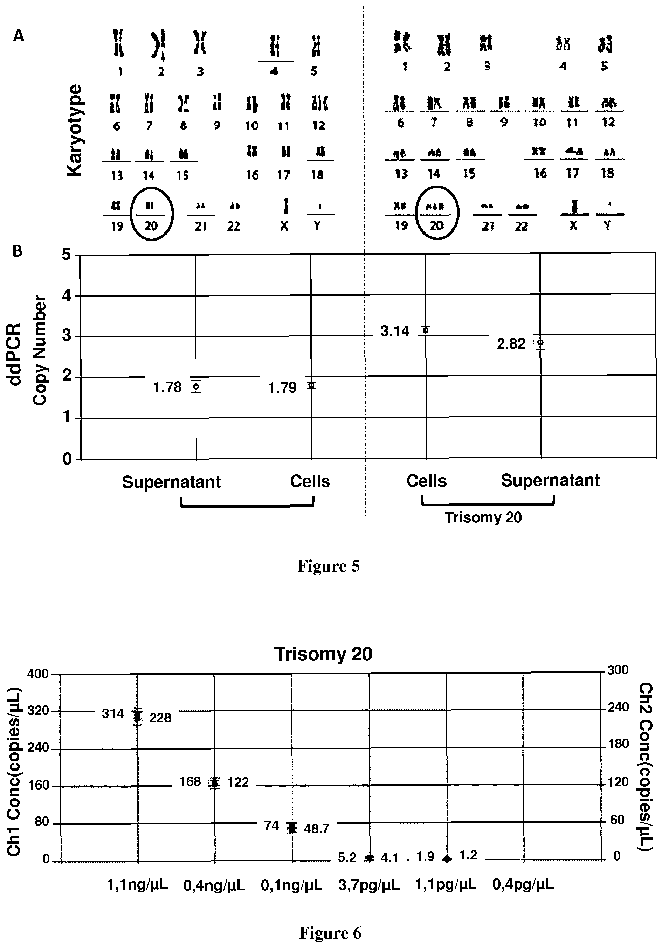

Assessing genetic integrity screening is possible by testing for cfDNA in hPSC-supernatant. We used two aneuploid human pluripotent stem cells (hPSC) lines HD129 and HD291 to validate the feasibility of the test. HD129 displayed a trisomy 20 (47, XY,+20), whereas HD291 displayed a trisomy 12 (47,XY,+12) as determined by conventional R-band karyotyping. Corresponding cells and supernatant were collected respectively for trisomy 20 analysis using our specific hyper-recurrent sequence and ddPCR approach. As shown in Figure 5, (i) the genomic aberration (in this case trisomy 20) is detected in the hPSC-supernatant using ddPCR approach for the HD129 hPSC line, but not in the HD291 line confirming the karyotype results, (ii) a correlation is found between genetic abnormalities screening result from supernatant and corresponding karyotype, demonstrating the proof of concept that cfNA present in the supernatant can be used to assess the genetic integrity of pluripotent stem cells. The advantage of stem cells screening by using supernatant would be the ability to evaluate stem cells genetic integrity without destruction. In addition, the use of this simple methodology, based on droplet digital polymerase chain reaction (ddPCR), enables the rapid, efficient and easy screening of hPSC lines from small quantities of material, including culture supernatent. These benefits may make this approach more attractive leading to potential utilization in routine. Finally, our method can be applied to any other experiments that require accurate analysis of the genome for genetic integrity testing (for example: Multipotent stem cells including such as Mesenchymal stem cells (MSC), germinal cells, Lymphocytes, embryos, or somatic cells).

**Minimum concentration of nucleic acids for robust test**

The sensitivity of trisomy 20 sequence detection using ddPCR was evaluated by testing different concentrations (1,1ng/$\mu$L, 0,4ng/$\mu$L, 0,1ng/$\mu$L, 3,7pg/$\mu$L, 1,1pg/$\mu$L, 0,4pg/$\mu$L) of nucleic acids extracted from supernatant collected from the hPSC line HD129. As shown in Figure 6, a trisomy 20 signal was detected between the signals obtained from very low concentration (as low as 0,1ng/$\mu$L) but still sufficient for a reliable screening result.

**REFERENCES:**

Throughout this application, various references describe the state of the art to which this invention pertains.

Abyzov, A., Mariani, J., Palejev, D., Zhang, Y., Haney, M. S., Tomasini, L., Ferrandino, A. F., Rosenberg Belmaker, L. A., Szekely, A., Wilson, M. et al. (2012). Somatic copy number mosaicism in human skin revealed by induced pluripotent stem cells. Nature 492, 438-42.

Cheng, L., Hansen, N. F., Zhao, L., Du, Y., Zou, C., Donovan, F. X., Chou, B. K., Zhou, G., Li, S., Dowey, S. N., Ye, Z., Chandrasekharappa, S. C., Yang, H., Mullikin, J. C., Liu, P. P., 2012. Low incidence of DNA sequence variation in human induced pluripotent stem cells generated by nonintegrating plasmid expression. Cell Stem Cell. 10, 337-44.

Choi, J. J., Reich, C. F., 3rd, Pisetsky, D. S., 2005. The role of macrophages in the in vitro generation of extracellular DNA from apoptotic and necrotic cells. Immunology. 115, 55-62.

Dan, S., Wang, W., Ren, J., Li, Y., Hu, H., Xu, Z., Lau, T. K., Xie, J., Zhao, W., Huang, H., Sun, L., Zhang, X., Liao, S., Qiang, R., Cao, J., Zhang, Q., Zhou, Y., Zhu, H., Zhong, M., Guo, Y., Lin, L., Gao, Z., Yao, H., Zhang, H., Zhao, L., Jiang, F., Chen, F., Jiang, H., Li, S., Wang, J., Duan, T., Su, Y., 2012. Clinical application of massively parallel sequencing-based prenatal noninvasive fetal trisomy test for trisomies 21 and 18 in 11,105 pregnancies with mixed risk factors. Prenat Diagn. 32, 1225-32.

Draper, J. S., Moore, H. D., Ruban, L. N., Gokhale, P. J., Andrews, P. W., 2004. Culture and characterization of human embryonic stem cells. Stem Cells Dev. 13, 325-36.

Fairbrother, G., Johnson, S., Musci, T. J., Song, K., 2013. Clinical experience of noninvasive prenatal testing with cell-free DNA for fetal trisomies 21, 18, and 13, in a general screening population. Prenat Diagn. 33, 580-3.

Funk, W. D., Labat, I., Sampathkumar, J., Gourraud, P. A., Oksenberg, J. R., Rosler, E., Steiger, D., Sheibani, N., Caillier, S., Stache-Crain, B., Johnson, J. A., Meisner, L., Lacher, M. D., Chapman, K. B., Park, M. J., Shin, K. J., Drmanac, R., West, M. D., 2012. Evaluating the genomic and sequence integrity of human ES cell lines; comparison to normal genomes. Stem Cell Res. 8, 154-64.

Gahan, P. B., Anker, P., Stroun, M., 2008. Metabolic DNA as the origin of spontaneously released DNA? Ann N Y Acad Sci. 1137, 7-17.

Ge, H., Huang, X., Li, X., Chen, S., Zheng, J., Jiang, H., Zhang, C., Pan, X., Guo, J., Chen, F., Chen, N., Fang, Q., Wang, W., 2013. Noninvasive prenatal detection for pathogenic CNVs: the application in alpha-thalassemia. PLoS One. 8, e67464.

Gore, A., Li, Z., Fung, H. L., Young, J. E., Agarwal, S., Antosiewicz-Bourget, J., Canto, I., Giorgetti, A., Israel, M. A., Kiskinis, E., Lee, J. H., Loh, Y. H., Manos, P. D., Montserrat, N., Panopoulos, A. D., Ruiz, S., Wilbert, M. L., Yu, J., Kirkness, E. F., Izpisua Belmonte, J. C., Rossi, D. J., Thomson, J. A., Eggan, K., Daley, G. Q., Goldstein, L. S., Zhang, K., 2011. Somatic coding mutations in human induced pluripotent stem cells. Nature. 471, 63-7.

Hui, L., Bianchi, D. W., 2013. Recent advances in the prenatal interrogation of the human fetal genome. Trends Genet. 29, 84-91.

Hussein, S. M., Batada, N. N., Vuoristo, S., Ching, R. W., Autio, R., Narva, E., Ng, S., Sourour, M., Hamalainen, R., Olsson, C., Lundin, K., Mikkola, M., Trokovic, R., Peitz, M., Brustle, O., Bazett-Jones, D. P., Alitalo, K., Lahesmaa, R., Nagy, A., Otonkoski, T., 2011. Copy number variation and selection during reprogramming to pluripotency. Nature. 471, 58-62.

Ji, J., Ng, S. H., Sharma, V., Neculai, D., Hussein, S., Sam, M., Trinh, Q., Church, G. M., McPherson, J. D., Nagy, A., Batada, N. N., 2012. Elevated coding mutation rate during the reprogramming of human somatic cells into induced pluripotent stem cells. Stem Cells. 30, 435-40.

Laurent, L. C., Ulitsky, I., Slavin, I., Tran, H., Schork, A., Morey, R., Lynch, C., Harness, J. V., Lee, S., Barrero, M. J., Ku, S., Martynova, M., Semechkin, R., Galat, V., Gottesfeld, J., Izpisua Belmonte, J. C., Murry, C., Keirstead, H. S., Park, H. S., Schmidt, U., Laslett, A. L., Muller, F. J., Nievergelt, C. M., Shamir, R., Loring, J. F., 2011. Dynamic changes in the copy number of pluripotency and cell proliferation genes in human ESCs and iPSCs during reprogramming and time in culture. Cell Stem Cell. 8, 106-18.

Lefort, N., Feyeux, M., Bas, C., Feraud, O., Bennaceur-Griscelli, A., Tachdjian, G., Peschanski, M., Perrier, A. L., 2008. Human embryonic stem cells reveal recurrent genomic instability at 20q11.21. Nat Biotechnol. 26, 1364-6.

Martins-Taylor, K., Xu, R. H., 2010. Determinants of pluripotency: from avian, rodents, to primates. J Cell Biochem. 109, 16-25.

Narva, E., Autio, R., Rahkonen, N., Kong, L., Harrison, N., Kitsberg, D., Borghese, L., Itskovitz-Eldor, J., Rasool, O., Dvorak, P., Hovatta, O., Otonkoski, T., Tuuri, T., Cui, W., Brustle, O., Baker, D., Maltby, E., Moore, H. D., Benvenisty, N., Andrews, P. W., Yli-Harja, O., Lahesmaa, R., 2010. High-resolution DNA analysis of human embryonic stem cell lines reveals culture-induced copy number changes and loss of heterozygosity. Nat Biotechnol. 28, 371-7.

Nicolaides, K. H., Syngelaki, A., Poon, L. C., Gil, M. M., Wright, D., 2014. Firsttrimester contingent screening for trisomies 21, 18 and 13 by biomarkers and maternal blood cell-free DNA testing. Fetal Diagn Ther. 35, 185-92.

Pasi, C. E., Dereli-Oz, A., Negrini, S., Friedli, M., Fragola, G., Lombardo, A., Van Houwe, G., Naldini, L., Casola, S., Testa, G., Trono, D., Pelicci, P. G., Halazonetis, T. D., 2011. Genomic instability in induced stem cells. Cell Death Differ. 18, 745-53.

Spits, C., Mateizel, I., Geens, M., Mertzanidou, A., Staessen, C., Vandeskelde, Y., Van der Elst, J., Liebaers, I., Sermon, K., 2008. Recurrent chromosomal abnormalities in human embryonic stem cells. Nat Biotechnol. 26, 1361-3.

Stroun, M., Lyautey, J., Lederrey, C., Olson-Sand, A., Anker, P., 2001. About the possible origin and mechanism of circulating DNA apoptosis and active DNA release. Clin Chim Acta. 313, 139-42.

Taapken, S. M., Nisler, B. S., Newton, M. A., Sampsell-Barron, T. L., Leonhard, K. A., McIntire, E. M., Montgomery, K. D., 2011. Karotypic abnormalities in human induced pluripotent stem cells and embryonic stem cells. Nat Biotechnol. 29, 313-4.

Umetani, N., Kim, J., Hiramatsu, S., Reber, H. A., Hines, O. J., Bilchik, A. J., Hoon, D. S., 2006. Increased integrity of free circulating DNA in sera of patients with colorectal or periampullary cancer: direct quantitative PCR for ALU repeats. Clin Chem. 52, 1062-9.

## Claims

1. An *in vitro* non invasive method for determining the quality of human pluripotent stem cell or of a differentiated cell derived therefrom comprising the steps of: i) providing a culture sample where the stem cell is grown, ii) extracting nucleic acids from the culture supernatant and iii) determining the presence and/or level of at least one genetic abnormality in the nucleic acid extraction, wherein said step iii) comprises the steps of

   i) determining the presence of at least one hyper-recurrent sequence selected from table 1 in the nucleic acid extraction, and ii) concluding that the pluripotent stem cells bears a genetic abnormality when at least one hyper-recurrent sequence is detected.

2. The method of claim 1, wherein said differentiated cell derived from the human pluripotent stem cell is selected in the group consisting of cardiomyocytes and progenitors thereof; neural progenitor cells; pancreatic islet cells, particularly pancreatic β-cells; hematopoietic stem and progenitor cells; mesenchymal stem cells; and muscle satellite cells.

3. The method of claim 1, wherein said differentiated cell derived from the human pluripotent stem cell is a lymphocyte.

4. The method of anyone of claims 1 to 3, wherein the genetic abnormality is detected by PCR.

5. The method of claim 4, wherein said PCR is digital droplet PCR.

6. The method of anyone of claims 1 to 3, wherein the genetic abnormality is detected by by next generation sequencing.

7. The method of anyone of claims 1 to 3, wherein the genetic abnormality is detected by probes arrays analysis.

8. A human pluripotent stem cell or a differentiated cell derived therefrom free from genetic abnormalities, for use in a transplantation method, wherein the said method comprises the steps of: i) performing the method according to any one of claims 1 to 5, ii) selecting human pluripotent stem cell free from genetic abnormalities, and iii) administering the pluripotent stem cell selected at step ii) or differentiated cells derived therefrom to a subject in need of regenerative treatment.

9. A human pluripotent stem cell or a differentiated cell derived therefrom free from genetic abnormalities, for use in a method for treating a disease in a subject in need of regenerative treatment, wherein the said method comprises the steps of: i) performing the method according to any one of claims 1 to 5, ii) selecting pluripotent stem cell free from genetic abnormalities, and iii) administering the human pluripotent stem cell selected at step ii) or differentiated cells derived therefrom to said subject.

**Patentansprüche**

1. Nichtinvasives *In-vitro*-Verfahren zur Bestimmung der Qualität einer humanen pluripotenten Stammzelle oder einer differenzierten Zelle, die davon abgeleitet ist, umfassend die Schritte: i) Bereitstellen einer Kulturprobe, in der die Stammzelle gezüchtet wird, ii) Extrahieren von Nukleinsäuren aus dem Kulturüberstand und iii) Bestimmen des Vorliegens und/oder des Ausmaßes von mindestens einer genetischen Anomalie in der Nukleinsäureextraktion, wobei der Schritt iii) die Schritte umfasst

   i) Bestimmen des Vorliegens von mindestens einer hyperrekurrierenden Sequenz, die aus Tabelle 1 ausgewählt ist, in der Nukleinsäureextraktion und ii) Folgern, dass die pluripotenten Stammzellen eine genetische Anomalie tragen, wenn mindestens eine hyperrekurrierende Sequenz nachgewiesen wird.

2. Verfahren nach Anspruch 1, wobei die differenzierte Zelle, die von der humanen pluripotenten Stammzelle abgeleitet ist, in der Gruppe bestehend aus Kardiomyozyten und Progenitoren davon; neuralen Progenitorzellen; pankreatischen Inselzellen, insbesondere pankreatischen β-Zellen; hämatopoetischen Stamm- und Progenitorzellen; mesenchymalen Stammzellen und Muskelsatellitenzellen ausgewählt ist.

3. Verfahren nach Anspruch 1, wobei die differenzierte Zelle, die von der humanen pluripotenten Stammzelle abgeleitet ist, ein Lymphozyt ist.

4. Verfahren nach einem der Ansprüche 1 bis 3, wobei die genetische Anomalie mittels PCR nachgewiesen wird.

5. Verfahren nach Anspruch 4, wobei die PCR eine digitale Tröpfchen-PCR ist.

6. Verfahren nach einem der Ansprüche 1 bis 3, wobei die genetische Anomalie mittels Next-Generation-Sequenzierung nachgewiesen wird.

7. Verfahren nach einem der Ansprüche 1 bis 3, wobei die genetische Anomalie mittels Sonden-Array-Analyse nachgewiesen wird.

8. Humane pluripotente Stammzelle oder davon abgeleitete differenzierte Zelle, die frei von genetischen Anomalien ist, zur Verwendung in einem Transplantationsverfahren, wobei das Verfahren die Schritte umfasst: i) Durchführen des Verfahrens nach einem der Ansprüche 1 bis 5, ii) Auswählen einer humanen pluripotenten Stammzelle, die frei von genetischen Anomalien ist, und iii) Verabreichen der in Schritt ii) ausgewählten pluripotenten Stammzelle oder von davon abgeleiteten differenzierten Zellen an einen Probanden, der einer regenerativen Behandlung bedarf.

9. Humane pluripotente Stammzelle oder davon abgeleitete differenzierte Zelle, die frei von genetischen Anomalien ist, zur Verwendung in einem Verfahren zur Behandlung einer Krankheit bei einem Probanden, der einer regenerativen Behandlung bedarf, wobei das Verfahren die Schritte umfasst: i) Durchführen des Verfahrens nach einem der Ansprüche 1 bis 5, ii) Auswählen einer pluripotenten Stammzelle, die frei von genetischen Anomalien ist, und iii) Verabreichen der in Schritt ii) ausgewählten humanen pluripotenten Stammzelle oder von davon abgeleiteten differenzierten Zellen an den Probanden.

**Revendications**

1. Méthode non invasive *in vitro* de détermination de la qualité d'une cellule souche pluripotente humaine ou d'une cellule différenciée dérivée de celle-ci comprenant les étapes : i) de fourniture d'un échantillon de culture où la cellule souche est cultivée, ii) d'extraction des acides nucléiques du surnageant de culture et iii) de détermination de la présence et/ou du niveau d'au moins une anomalie génétique dans l'extraction d'acides nucléiques, dans laquelle ladite étape iii) comprend les étapes de

   i) détermination de la présence d'au moins une séquence hyper-récurrente sélectionnée dans le tableau 1 dans l'extraction d'acides nucléiques, et ii) conclusion que les cellules souches pluripotentes portent une anomalie génétique lorsqu'au moins une séquence hyper-récurrente est détectée.

2. Méthode selon la revendication 1, dans laquelle ladite cellule différenciée dérivée de la cellule souche pluripotente humaine est sélectionnée dans le groupe consistant en des cardiomyocytes et des progéniteurs de celle-ci ; des cellules progénitrices neuronales ; des cellules des îlots pancréatiques, notamment des cellules β pancréatiques ; des cellules progénitrices et souches hématopoïétiques ; des cellules souches mésenchymateuses ; et des cellules satellites musculaires.

3. Méthode selon la revendication 1, dans laquelle ladite cellule différenciée dérivée de la cellule souche pluripotente humaine est un lymphocyte.

4. Méthode selon l'une quelconque des revendications 1 à 3, dans laquelle l'anomalie génétique est détectée par PCR.

5. Méthode selon la revendication 4, dans laquelle ladite PCR est une PCR numérique en gouttelette.

6. Méthode selon l'une quelconque des revendications 1 à 3, dans laquelle l'anomalie génétique est détectée par séquençage de nouvelle génération.

7. Méthode selon l'une quelconque des revendications 1 à 3, dans laquelle l'anomalie génétique est détectée par analyse de jeux de sondes.

8. Cellule souche pluripotente humaine ou cellule différenciée dérivée de celle-ci dépourvue d'anomalies génétiques, pour son utilisation dans une méthode de transplantation, dans laquelle ladite méthode comprend les étapes : i) de réalisation de la méthode selon l'une quelconque des revendications 1 à 5, ii) de sélection d'une cellule souche pluripotente humaine dépourvue d'anomalies génétiques, et iii) d'administration de la cellule souche pluripotente sélectionnée à l'étape ii) ou de cellules différenciées dérivées de celle-ci à un sujet nécessitant un traitement régénératif.

9. Cellule souche pluripotente humaine ou cellule différenciée dérivée de celle-ci dépourvue d'anomalies génétiques, pour son utilisation dans une méthode de traitement d'une maladie chez un sujet nécessitant un traitement régénératif, dans laquelle ladite méthode comprend les étapes : i) de réalisation de la méthode selon l'une quelconque des revendications 1 à 5 ; ii) de sélection d'une cellule souche pluripotente dépourvue d'anomalies génétiques, et iii) d'administration de la cellule souche pluripotente sélectionnée à l'étape ii) ou de cellules différenciées dérivées de celle-ci audit sujet.

Figure 1

Genome position

Figure 2

Figure 3

Figure 4

**A**

Karyotype

**B**

Figure 5

Trisomy 20

Figure 6

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2005021786 A
- US 5622824 A
- US 6140053 A
- US 4683202 A
- US 5854033 A

**Non-patent literature cited in the description**

- **KRISTEN MARTINS-TAYLOR et al.** *NATURE BIO-TECHNOLOGY,* 01 June 2011, vol. 29 (6), 488-491
- **BENTLEY et al.** *Nature,* 2008, vol. 456, 53-59
- **BARANY.** *Proc. Natl. Acad. Sci. USA,* 1991, vol. 88, 189-193
- **GUATELLI et al.** *Proc. Natl. Acad. Sci. USA,* 1990, vol. 57, 1874-1878
- **KWOH et al.** *Proc. Natl. Acad. Sci. USA,* 1989, vol. 86, 1173-1177
- **LIZARDI et al.** *Biol. Technology,* 1988, vol. 6, 1197
- **ABYZOV, A. ; MARIANI, J. ; PALEJEV, D. ; ZHANG, Y. ; HANEY, M. S. ; TOMASINI, L. ; FERRANDINO, A. F. ; ROSENBERG BELMAKER, L. A. ; SZEKELY, A. ; WILSON, M. et al.** Somatic copy number mosaicism in human skin revealed by induced pluripotent stem cells. *Nature,* 2012, vol. 492, 438-42
- **CHENG, L. ; HANSEN, N. F. ; ZHAO, L. ; DU, Y. ; ZOU, C. ; DONOVAN, F. X. ; CHOU, B. K. ; ZHOU, G. ; LI, S. ; DOWEY, S. N.** Low incidence of DNA sequence variation in human induced pluripotent stem cells generated by nonintegrating plasmid expression. *Cell Stem Cell,* 2012, vol. 10, 337-44
- **CHOI, J. J. ; REICH, C. F. ; PISETSKY, D. S.** The role of macrophages in the in vitro generation of extracellular DNA from apoptotic and necrotic cells. *Immunology,* 2005, vol. 115, 55-62
- **DAN, S. ; WANG, W. ; REN, J. ; LI, Y. ; HU, H. ; XU, Z. ; LAU, T. K. ; XIE, J. ; ZHAO, W. ; HUANG, H.** Clinical application of massively parallel sequencing-based prenatal noninvasive fetal trisomy test for trisomies 21 and 18 in 11,105 pregnancies with mixed risk factors. *Prenat Diagn.,* 2012, vol. 32, 1225-32
- **DRAPER, J. S. ; MOORE, H. D. ; RUBAN, L. N. ; GOKHALE, P. J. ; ANDREWS, P. W.** Culture and characterization of human embryonic stem cells. *Stem Cells Dev.,* 2004, vol. 13, 325-36
- **FAIRBROTHER, G. ; JOHNSON, S. ; MUSCI, T. J. ; SONG, K.** Clinical experience of noninvasive prenatal testing with cell-free DNA for fetal trisomies 21, 18, and 13, in a general screening population. *Prenat Diagn.,* 2013, vol. 33, 580-3
- **FUNK, W. D. ; LABAT, I. ; SAMPATHKUMAR, J. ; GOURRAUD, P. A. ; OKSENBERG, J. R. ; ROSLER, E. ; STEIGER, D. ; SHEIBANI, N. ; CAILLIER, S. ; STACHE-CRAIN, B.** Evaluating the genomic and sequence integrity of human ES cell lines; comparison to normal genomes. *Stem Cell Res.,* 2012, vol. 8, 154-64
- **GAHAN, P. B. ; ANKER, P. ; STROUN, M.** Metabolic DNA as the origin of spontaneously released DNA?. *Ann N Y Acad Sci.,* 2008, vol. 1137, 7-17
- **GE, H. ; HUANG, X. ; LI, X. ; CHEN, S. ; ZHENG, J. ; JIANG, H. ; ZHANG, C. ; PAN, X. ; GUO, J. ; CHEN, F.** Noninvasive prenatal detection for pathogenic CNVs: the application in alpha-thalassemia. *PLoS One,* 2013, vol. 8, e67464
- **GORE, A. ; LI, Z. ; FUNG, H. L. ; YOUNG, J. E. ; AGARWAL, S. ; ANTOSIEWICZ-BOURGET, J. ; CANTO, I. ; GIORGETTI, A. ; ISRAEL, M. A. ; KISKINIS, E.** Somatic coding mutations in human induced pluripotent stem cells. *Nature,* 2011, vol. 471, 63-7
- **HUI, L. ; BIANCHI, D. W.** Recent advances in the prenatal interrogation of the human fetal genome. *Trends Genet.,* 2013, vol. 29, 84-91
- **HUSSEIN, S. M. ; BATADA, N. N. ; VUORISTO, S. ; CHING, R. W. ; AUTIO, R. ; NARVA, E. ; NG, S. ; SOUROUR, M. ; HAMALAINEN, R. ; OLSSON, C.** Copy number variation and selection during reprogramming to pluripotency. *Nature,* 2011, vol. 471, 58-62
- **JI, J. ; NG, S. H. ; SHARMA, V. ; NECULAI, D. ; HUSSEIN, S. ; SAM, M. ; TRINH, Q. ; CHURCH, G. M. ; MCPHERSON, J. D. ; NAGY, A.** Elevated coding mutation rate during the reprogramming of human somatic cells into induced pluripotent stem cells. *Stem Cells,* 2012, vol. 30, 435-40

- **LAURENT, L. C. ; ULITSKY, I. ; SLAVIN, I. ; TRAN, H. ; SCHORK, A. ; MOREY, R. ; LYNCH, C. ; HARNESS, J. V. ; LEE, S. ; BARRERO, M. J.** Dynamic changes in the copy number of pluripotency and cell proliferation genes in human ESCs and iPSCs during reprogramming and time in culture. *Cell Stem Cell,* 2011, vol. 8, 106-18

- **LEFORT, N. ; FEYEUX, M. ; BAS, C. ; FERAUD, O. ; BENNACEUR-GRISCELLI, A. ; TACHDJIAN, G. ; PESCHANSKI, M. ; PERRIER, A. L.** Human embryonic stem cells reveal recurrent genomic instability at 20q11.21. *Nat Biotechnol.,* 2008, vol. 26, 1364-6

- **MARTINS-TAYLOR, K. ; XU, R. H.** Determinants of pluripotency: from avian, rodents, to primates. *J Cell Biochem.,* 2010, vol. 109, 16-25

- **NARVA, E. ; AUTIO, R. ; RAHKONEN, N. ; KONG, L. ; HARRISON, N. ; KITSBERG, D. ; BORGHESE, L. ; ITSKOVITZ-ELDOR, J. ; RASOOL, O. ; DVORAK, P.** High-resolution DNA analysis of human embryonic stem cell lines reveals culture-induced copy number changes and loss of heterozygosity. *Nat Biotechnol.,* 2010, vol. 28, 371-7

- **NICOLAIDES, K. H. ; SYNGELAKI, A. ; POON, L. C. ; GIL, M. M. ; WRIGHT, D.** Firsttrimester contingent screening for trisomies 21, 18 and 13 by biomarkers and maternal blood cell-free DNA testing. *Fetal Diagn Ther.,* 2014, vol. 35, 185-92

- **PASI, C. E. ; DERELI-OZ, A. ; NEGRINI, S. ; FRIEDLI, M. ; FRAGOLA, G. ; LOMBARDO, A. ; VAN HOUWE, G. ; NALDINI, L. ; CASOLA, S. ; TESTA, G.** Genomic instability in induced stem cells. *Cell Death Differ.,* 2011, vol. 18, 745-53

- **SPITS, C. ; MATEIZEL, I. ; GEENS, M. ; MERTZANIDOU, A. ; STAESSEN, C. ; VANDESKELDE, Y. ; VAN DER ELST, J. ; LIEBAERS, I. ; SERMON, K.** Recurrent chromosomal abnormalities in human embryonic stem cells. *Nat Biotechnol.,* 2008, vol. 26, 1361-3

- **STROUN, M. ; LYAUTEY, J. ; LEDERREY, C. ; OLSON-SAND, A. ; ANKER, P.** About the possible origin and mechanism of circulating DNA apoptosis and active DNA release. *Clin Chim Acta.,* 2001, vol. 313, 139-42

- **TAAPKEN, S. M. ; NISLER, B. S. ; NEWTON, M. A. ; SAMPSELL-BARRON, T. L. ; LEONHARD, K. A. ; MCINTIRE, E. M. ; MONTGOMERY, K. D.** Karotypic abnormalities in human induced pluripotent stem cells and embryonic stem cells. *Nat Biotechnol.,* 2011, vol. 29, 313-4

- **UMETANI, N. ; KIM, J. ; HIRAMATSU, S. ; REBER, H. A. ; HINES, O. J. ; BILCHIK, A. J. ; HOON, D. S.** Increased integrity of free circulating DNA in sera of patients with colorectal or periampullary cancer: direct quantitative PCR for ALU repeats. *Clin Chem.,* 2006, vol. 52, 1062-9